# EUROPEAN PATENT SPECIFICATION

(11) **EP 1 331 932 B1**
(45) Date of publication and mention of the grant of the patent: **04.04.2007**
(21) Application number: 01983374.8
(22) Date of filing: 08.11.2001
(51) Int. Cl.: A61K 31/537, A61P 31/18

(54) **USE OF XESTOSPONGIN C FOR TREATING OR PREVENTING HIV INFECTION**
VERWENDUNG VON XESTOSPONGIN C ZUR BEHANDLUNG ODER VORBEUGUNG EINER HIV-INFEKTION
UTILISATION DE LA XESTOSPONGINE C POUR LE TRAITEMENT OU LA PRÉVENTION D'UNE INFECTION À VIH

(30) Priority: 09.11.2000 US 246763 P; 13.06.2001 US 878918; 27.07.2001 US 307860 P
(43) Date of publication of application: 06.08.2003
(73) Proprietor: Contrimmune Biotechnology Inc., Winnipeg, Manitoba R3B 3C5 (CA)
(72) Inventor: GLAZNER, Gordon, Winnipeg, Manitoba R2H 2A6 (CA)
(74) Representative: Tombling, Adrian George
(86) International application number: PCT/CA2001/001575
(87) International publication number: WO 2002/038140

(56) References cited:
- EP-A- 0 467 185
- EP-A1- 0 355 905
- EP-A1- 0 520 372
- EP-A2- 0 992 587
- WO-A1-94/07507
- WO-A2-97/04783
- US-A- 6 027 731
- DATABASE WPI Week 198506, 21 December 1984 Derwent Publications Ltd., London, GB; AN 1985-034355, XP002204006 NAKAGAWA TADASHI ET AL: '1-Oxaquinolizidine' & JP 59 227 885 A (SUNTORY LTD) 21 December 1984 & PATENT ABSTRACTS OF JAPAN vol. 009, no. 096 (C-278), 25 April 1985 (1985-04-25) & JP 59 227885 A (SUNTORY KK), 21 December 1984 (1984-12-21)
- GAFNI J. ET AL: 'XESTOSPONGINS: POTENT MEMBRANE PERMEABLE BLOCKERS OF THE INOSITOL 1,4,5-TRISPHOSPHATE RECEPTOR' NEURON, CAMBRIDGE, MA, US vol. 3, no. 19, September 1997, pages 723 - 733, XP001077738
- DATABASE BIOSIS [Online] BIOSCIENCES INFORMATION SERVICE, PHILADELPHIA, PA, US; 1993 BENTLEY NIHCOLAS ET AL: "The synthesis of 1-oxaquinolizidines via the mercury(II) acetate mediated cyclization of piperidine alcohols." Database accession no. PREV199396040934 XP002204005 & TETRAHEDRON, vol. 49, no. 20, 1993, pages 4315-4320, XP001073942 ISSN: 0040-4020
- MAYNE M. ET AL: 'Release of calcium from inositol 1,4,5-trisphosphate receptor-regulated stores by HIV-1 Tat regulates TNF-alpha production in human macrophages.' JOURNAL OF IMMUNOLOGY (BALTIMORE, MD.: 1950) UNITED STATES 15 JUN 2000 vol. 164, no. 12, 15 June 2000, pages 6538 - 6542, XP002204004 ISSN: 0022-1767
- FAUCI A.S.: "Host factors and the pathogenesis of HIV-induced disease" NATURE, vol. 384, 1996, pages 529-534,

## Description

### FIELD OF THE INVENTION

The present invention relates generally to the field of therapeutic compounds for preventing or ameliorating viral diseases.

### BACKGROUND OF THE INVENTION

The transcription factor nuclear factor κB (NF-κB) is a member of the NF-κB/Rel family which includes p50, p52, p65 (Rel A), c-Rel and Rel B proteins. Prototypical NF-κB is a p50-p65 heterodimer that is usually retained in the cytoplasm of unstimulated cells in an inactive form as part of a complex of inhibitory proteins (IκB). In response to various stimuli, the inhibitory protein is phosphorylated, rapidly ubiquitinated and subsequently proteolyzed by a 26 S proteasome complex. The degradation of IκB unmasks the nuclear localization signal of the NF-κB heterodimer which then translocates into the nucleus where it binds to its cognate sequence to regulate gene transcription (Baeuerle and Baltimore, 1996, *Cell* **87**:13-20; Mattson, 1998, *Int. Rev. Neurobiol.* **42**:103-168).

In the nervous system NF-κB is expressed in many cell types and is constitutively active in subsets of cells in rodent cortex and hippocampus (Kaltschmidt et al., 1994, *Mol. Cell. Biol.* **14**:3981-92). In neurons, NF-κB activity is elevated after seizure activity (Prasad et al., 1994 *Neurosci. Lett.* **170**:145-148; Rong and Baudry, 1996, *J*. *Neurochem.* **67**:662-668) global (Clemens et al., 1997, *Brain Res. Mol. Brain Res.* **48**:187-96) and focal (Schneider et al., 1999, *Nat. Med* **5**:554-559) ischemia, and in Alzheimer's (Kaltschmidt, 1997, *Proc. Natl. Acad. Sci. USA* **94**:2642-2647; Akama et al., 1998, *Proc. Natl. Acad. Sci. USA* **95**:5795-5800) and Parkinson's diseases (Hunot et al., 1997, *Proc. Natl. Acad. Sci. USA* **94**:7531-7536). Because activation of NF-κB has been associated with cell injury and death in different pathological states, some investigators have proposed that this transcription factor contributes to the cell death process (Grilli et al., 1996, *Science* **274**:1383-1385; Clemens et al.,1997). However, results from cell culture and *in vivo* studies have demonstrated that activation of NF-κB represents a highly protective response in neurons (Barger et al., 1995, *Proc. Natl. Acad. Sci. USA.* **92**:9328-9332; Mattson et al., 1997, *J. Neurosci. Res.* **49**:681-697; Taglialatela et al., 1997, *J*. *Neurosci. Res.* **47**:155-156; Yu et al., 1999, *J. Neurosci.* **19**:8856-8865). The neuroprotective role played by NF-κB involves the ability to induce the expression of genes encoding anti-oxidant (manganese superoxide dismutase) (Mattson et al., 1997), calcium stabilizing (Calbindin D28K) (Cheng et al., 1994, *Neuron* **12**:139-153), and anti-apoptotic proteins (Bcl-2) (Tamatani et al., 1999, *J. Biol. Chem.* **274**:8531-8538). A number of ligands have been shown to activate NF-κB, including tumor necrosis factor α (TNFα) (Barger et al., 1995; Hazan et al., 1990, *Proc. Natl. Acad. Sci. USA* **87**:7861-7865; Swingler et al., 1992, *AIDS Res. Hum. Retroviruses* **8**:487-493), IL1α (Nonaka and Huang, 1990, *Mol. Cell. Biol.* **10**:6283-6289), glutamate (Kaltschmidt et al., 1995, *Proc. Natl. Acad. Sci. USA* **92**:9618-9622), nerve growth factor (Carter et al., 1996, *Science* **272**:542-545; Maggirwar et al., 1998, *J*. *Neurosci.* **18**:10356-10365), and secreted amyloid precursor protein (Barger and Harmon, 1997, *Nature* **388**:878-881). In addition, NF-κB is highly inducible by cellular stress: enhanced activity has been associated with elevated levels of oxidation, alterations in calcium homeostasis, and DNA damage (Mercurio and Manning, 1999, *Oncogene* **18**:6163-6171; Mattson et al., 2000, *J. Neurochem.* **74**:443-456; Gius et al., 1999, *Toxicol. Lett.* **106**:93-106).

In the central nervous system, changes in the concentration of intracellular calcium [Ca²⁺]ᵢ affect numerous functions including neurotransmitter release, and long-term potentiation and depression (Kennedy, 1989, *Trends Neurosci.* **12**:417-420; Berridge, 1998, *Neuron* **21**:13-26). However, the significance of Ca²⁺ released from internal stores such as endoplasmic reticulum (ER) has become increasingly apparent. In neurons, the ER is a continuous network of intracellular tubules and cisternae distributed throughout the cell and represents a large and releasable pool of intracellular calcium.

Regardless of its origin, increased [Ca²⁺]ᵢ often leads to the release of Ca²⁺ stored in the ER, a phenomenon referred to as calcium-dependent calcium release (Verkhratsky and Shmigol, 1996, *Cell Calcium* **19**:1-14). This phenomenon is regulated by the ER-resident IP₃ (inositol-1,4,5 triphosphate) and ryanodine receptor calcium channels. The intracellular signaling molecule IP₃, the ligand for IP₃ receptors, is generated by activation of phospholipase C and cleavage of phosphoinositol bisphosphate into diacylglycerol and IP₃. Calcium regulates the sensitivity of these channels to IP₃, increasing the probability that the channel will be open (Berridge, 1998). Therefore, alterations in [Ca²⁺]ᵢ will affect the activity of the ER IP₃ receptor, and thus affect cellular events regulated by this receptor. The phosphoinositide system is particularly well developed in the brain (Nahorski, 1988, *Trends Neurosci.* **11**:444-448; Challiss et al., 1991, *Biochem. Soc. Trans.* **19**:888-893; Furuichi and Mikoshiba, 1995, *J. Neurochem.* **64**:953-960) and IP₃ receptors act as detectors that integrate information from neural signals (Berridge et al., 1998).

As will be appreciated by one knowledgeable in the art, the role and importance of calcium varies in different cell types. For example, as discussed herein, in neurons, there are calcium channels in the plasma membrane and, as discussed below, IP₃ receptors play a significant role. In cardiac cells, IP₃ is a minor player compared to the ryanodine receptor. Furthermore, in cardiac cells, calcium release is needed for contraction. It is of note that, as discussed below, there is no excitation-coupled release of calcium in immune cells.

Research into mechanisms of calcium signalling by the endoplasmic reticulum has thus followed two tracks: one involving excitation-contraction in muscle and the other calcium release by IP₃ and other cellular mediators. The specialized endoplasmic reticulum of muscle, the sarcoplasmic reticulum, has long been known to show the property of calcium-induced calcium release (Ebashi, 1991, *Ann. Rev. Physiol.* **53:** 1-16; Schneider, 1994, *Ann. Rev. Physiol.* **56**: 463-484). This finding led to the concept of 'trigger' calcium wherein a small increment of calcium triggers the all-or-none calcium-induced calcium release process. In the case of heart muscle as well as other excitable cell types, it has been hypothesized that the trigger calcium comes from calcium influx through voltage-activated calcium channels as a result of the automatic, paced cardiac action potential (Putney and Ribeiro, 2000, *Cell. Mol*. *Life Sci.* **57**: 1272-1286).

It has also been hypothesized that depletion of stored Ca²⁺ may signal apoptosis (Bian et al., 1997; *Am. J. Physiol.* **272**: C1241-C1249), although others have reported that partial reduction of endoplasmic reticulum calcium stores actually protected against apoptosis (Pinton et al., 2000, *J*. *Cell Biol.* **148**: 857-862).

Activation of transcription factor NF-κB is induced by a number of bacteria and viruses. The bacteria which induce NF-κB activity include, for example, EPEC, enteropathogenic *E*. *coli* (Savkovic et al., 1997, *Am. J. Physiol.* **273**:C1160-1167), *Gardnerella vaginalis* (Hashemi et al., 1999, *J. Infect. Dis.* **179**:924-930), *Helicobacter pylori* (Munzenmaier et al., 1997, *J. Immunol.* **159**:6140-6147), *Lactobacilli* (Klebanoff et al., 1999, *J. Infect. Dis.* **179**:653-660), *Listeria monocytogenes* (Hauf et al., 1994, *Infect. Immun.* **62**:2740-2747), *Micoplasma fermentans* (Marie et al., 1999, *Infect. Immun.* **67**:688-693), *Mycobaceria tuberculosis* (Zhang et al., 1994, *Proc. Natl. Acad. Sci. USA* **91**:2225-2229), *Neisseria gonorrhoeae* (Naumann et al., 1997, *J. Exp. Med.* **186**:247-258), *Rickettsia rickettsii* (Sporn et al., 1997, *Infect. Immun.* **65**:2786-2791), *Salmonella dublin* (Eaves-Pyles et al., 1999, *Infect. Immun.* **67**:800-804), *Salmonella typhimurium* (Hobbie et al., 1997, *J*. *Immunol.* **159**:5550-5559), *Shigella flexneri* (Dyer et al., 1993, *Infect. Immun.* **61**:4427-4433), *Staphylococcus aureus* (Busam et al., 1992, *Infect. Immun.* **60**:2008-2015). The viruses that induce NF-κB activity include for example, Human Immunodeficiency Virus (HIV) (Bachelerie et al., 1991, *Nature* **350**:709-712), Adenovirus (Shurman et al., 1989, *J. Immunol.* **143**:3806-3812), Epstein-Barr Virus (Hammarskjold and Simurda, 1992, *J*. *Virol.* **66**:6496-6501), Hepatitis B Virus (Siddiqui et al., 1989, *Virology* **169**:479-484), Cytomegalovirus (Sambucetti et al., 1989, *EMBO J.* **8**:4251-4258), HTLV-1 (Leung and Nabel, 1988, *Nature* **333**:776-778; Ballard et al., 1988, *Science* **241**:1652-1655), Influenza Virus (Ronni et al., 1997, *J*. *Immunol.* **158**:2363-2374), Measles Virus (Harcourt et al., 1999, *J*. *Med. Virol.* **57**:9-16), Molony Murine Leukemia Virus (Pak and Fuller, 1996, *J*. *Virol.* **70**:4167-4172), Newcastle Disease Virus (Ten et al., 1992, *EMBO J.* **11**:195-203), Respiratory Syncytial Virus (Mastronarde et al., 1996, *J*. *Infect. Dis.* **174**:262-267; Garofalo et al., 1996, *J*. *Virol.* **70**:8773-8781), Rhinovirus (Zhu et al., 1996, *J. Biol. Chem.* **271**:15815-15822; Zhu et al, 1996, *J*. *Clin. Invest.* **97**:421-430), Sendai paramyxo virus (Hiscott et al, 1989, *J*. *Virol.* **63**:2557-2566), Sindbis Virus (Lin et al., 1995, *J. Cell Biol.* **131:**1149-1161) and the Herpes Virus family, for example, Herpes Virus Saimiri (Yao et al., 1995, *Immunity* **3**:811-821), Human Herpes Virus 6 (Ensoli et al., 1989, *EMBO J* **8**:3019-3027), and Herpes Simplex Virus -1 (Gimble et al., 1988, *J*. *Virol.* **62**:4104-4112). A number of viruses have also evolved to include NF-κB binding sites within their promoters, for example, Hepatitis C Virus, Adenovirus (Williams et al., 1990, *EMBO J.* **9**:4435-4442), Avian Leukosis Virus (Bowers et al., 1996, *J*. *Virol.* **70**:3051-3059), Bovine Leukemia Virus (Brooks et al., 1995, *J*. *Virol.* **69**:6005-6009), Cytomegalovirus (Sambucetti et al., 1989), Epstein-Barr Virus (Sugano et al., 1997, *J*. *Exp. Med.* **186**:731-737), Feline Leukemia Virus, HIV (Nabel and Baltimore, 1987, *Nature* **326**:711-713; Griffin et al., 1989, *Nature* **339**:70-73), Herpes simplex virus (Rong et al., 1992, *Virology* **189**:750-756), Polyoma virus (Ranganathan and Khalili, 1993, *Nuc. Acids Res*. **21**:1959-1964), Measles virus (Harcourt et al., 1999), Simian immunodeficiency virus (Bellas et al., 1993, *J*. *Virol.* **67**:2908-2913) and Simian Virus 40 (Kanno et al., 1989, *EMBO J.* **8**:4205-4214). Thus, those viruses which both induce NF-κB activation and have NF-κB binding sites within their transcription promoters would in essence be inducing their own replication upon transfection. It is also important to note that it has been hypothesized that a low level of NF-κB activation is part of the mechanism by which some viruses, for example, Epstein-Barr virus, Herpes simplex virus, Cytomegalovirus or Human Immunodeficiency virus maintain their chronic infections (Pahl, 1999, *Oncogene* **18**:6853-6866).

Infection by the Human Immunodeficiency virus, in most cases, leads to the development of acquired immunodeficiency syndrome (AIDS). Specifically, the HIV virus particle binds to CD4-bearing cells, such as T-lymphocytes, monocytes and macrophages, and is internalized through a viral-envelope mediated fusion of viral and host cell membranes. Therein, the virus relies on the interaction of cellular and virus-encoded trans-acting factors to produce the products necessary in order to assemble new virus particles. That is, the virus corrupts the cellular machinery of infected cells by causing the cell to replicate copies of the HIV virus. It is of note that cellular factors required by HIV include transcription factors NF-κB and SP1. The infection leads to depletion of T-cells, which in turn causes immundeficiency, leaving the host susceptible to secondary infections, which often prove fatal.

Initially, transcription of HIV is slow and inefficient, meaning that replication of the viral genome does not occur at a high rate. However, some of the transcripts are translated, producing viral proteins, one of which is Tat. Tat returns to the nucleus and binds to a structure formed in the viral RNA during transcription known as TAR. Tat binding at TAR is believed to activate transcription by recruiting transcription factors, thereby increasing the rate of transcription and Tat also promotes production of full-length viral transcripts. Several other roles have been proposed for Tat in addition to its role as a transcriptional activator. For example, it has been shown that infected cells shed or release tat during the acute phase of HIV infection. It has therefore been hypothesized that tat enters non-infected cells and disrupts host immune function by activating a wide variety of genes regulated by specific viral and endogenous cellular promoters (Vaishnav and Wong-Stall, 1991, *Ann. Rev. Biochem.* **60**: 577; Kumar et al., 1998, *J. Immunol.* **161**: 776). It has also been proposed that tat renders uninfected cells susceptible to productive viral infection (Goldstein, 1996, *Nature Medicine* **9**: 960-964). Furthermore, it has been proposed that tat acts on uninfected brain-derived cells to cause NF-κB activation and neurotoxicity as well as act directly on neurons to cause excitotoxicity and cell death by apoptosis (Chen et al., 1997, *J*. *Biol. Chem.* **272:** 22385-22388).

As discussed above, the activation of NF-κB is tightly regulated by immune-activation, cytokine-activation and stress-activation pathways in CD4⁺ T cells and monocytes/macrophages, which are the cellular targets of HIV infection. Thus, NF-κB has been hypothesized to play a role in HIV pathogenesis, although it has also been noted that the plethora of regulatory cascades suggest that while pharmacologic approaches may be developed to modulate NF-κB activity, the role that this modulation could play in the treatment of HIV infection is unclear (Rabson and Lin, 2000, *Adv. Pharmacology* **48:** 161-207). However, these authors also noted that NF-κB can enhance HIV replication and can activate expression of HIV from latently infected cells, leading to the hypothesis that stimuli that induce NF-κB would lead to increased HIV replication and ultimately to a more rapid progression from asymptomatic HIV infection to AIDS.

PCT Application WO 99/02185 teaches the construction of a synthetic tat peptide and the use thereof for immunization against AIDS. It is also noted therein that tat protein is released extracellularly, making it available to be taken up by other infected cells to enhance transcription of HIV in the cells and to be taken up by non-infected cells, altering host cell gene activations and rendering the cells susceptible to infection by the virus. Thus, tat uptake by both infected and uninfected cells is important for infectivity of HIV. Based on this, it is stated that immunization of mammals to induce antibodies to HIV tat protein could be used as a potential AIDS vaccine.

PCT Application WO 00/78969 teaches HIV-1-tat-multiple peptide conjugates and the use of same to induce an immune response. It is also noted therein that extracellular tat causes activation of intracellular signal transduction pathways that culminate in the production of various cytokines. Furthermore, it is proposed that one mechanism for viral activation by tat is the TAR-independent activation of virus replication involving the host factor NF-κB by an intracellular signal transduction pathway.

US Patent 6,024,965 teaches the use of cytotoxic T-cell epitopes of the Rev and/or Tat protein for stimulating a specific cytotoxic T-cell response in a host. This is based on the observation that the presence of cytotoxic T-cells to Rev and/or Tat in samples of a subject infected with HIV is an indication of a stable disease condition and a favourable prognosis of lack of progression to disease.

US Patent 5,821,046 teaches a synthetic RNA molecule arranged to bind tat protein. The use of the RNA molecule as a therapeutic for inhibiting HIV is also taught. Similarly, US Patent 5,637,461 teaches nucleic acid molecules for binding Tat protein.

US Patent 5,606,026 teaches the use of isolated natural lgM antibody in diagnosis of AIDS. The use of the low-affinity natural serum lgM to monitor the efficacy of therapeutic treatments is also taught. Furthermore, the idea that entry of Tat into resting, non-productive HIV infected cells activates these cells to produce virus is also disclosed.

A number of studies have shown that impairment of ER function with accumulation of proteins in the ER and the consequent release of Ca²⁺ from this organelle causes activation of NF-κB and NF-κB-dependent gene expression (Pahl and Baeuerle, 1995, *EMBO J.* **14**:2580-2588; Pahl and Baeuerle, 1997, *Trends Biochem. Sci.* **22**:63-7).

Xestospongin C (XeC) is a compound isolated from *Xestospongia* species. The use of XeC to block the inositol 1,4,5-triphosphate (IP₃) receptor was first described by Gafni et al. (Gafni et al., 1997, *Neuron* **19**:723-733): It has also been shown that XeC decreased the frequency of calcium oscillations in cardiac cells which was in turn linked to a parallel decrease in NF-κB activity (Hu et al., 1999, *J*. *Biol. Chem.* **274**:33995-33998). It was assumed that this established a direct link between calcium oscillation frequency and the activity of NF-κB during agonist (histamine) stimulation. It is of note that this observation was held to be consistent with the prior art teachings, that is, that activation of NF-κB is calcium-dependent, as discussed above. It is also important to note that these experiments were carried out in cardiac cells, wherein the majority of the calcium within the endoplasmic reticulum of these cells is regulated by the ryanodine receptor calcium channels, as the ryanodine receptors greatly outnumber the IP₃ receptors in cardiac cells. However, in other cell types, such as cells of the immune system and neurons, this is in fact reversed, with the vast majority of the calcium being regulated by the IP₃ receptors. Furthermore, the authors argued that calcium oscillation was important specifically in excitable cells (i.e. neurons and muscle cells) due to the calcium link. It is of note that no mention is made of possible effects on immune cells, nor is it obvious at all that the regulation in cardiac cells would be equivalent, or even related, to regulation in immune cells, as discussed above. It is also of note that the concentration of XeC required to observe this effect in cardiac cells is very high, which suggests that the effect is not entirely due to the IP₃ receptors but also due to binding of XeC at the ryanodine receptors.

In addition, therapies that target the IP₃ pathway have been proposed to be beneficial in the treatment of HIV dementia (Mayne et al., 2000, *J. Immunol.* **164**:6538-6542), based on the observation that calcium release from IP₃ receptor regulated stores of calcium initiated tat-induced production of TNF-α.

NF-κB is the major nuclear regulator of the inflammatory response in humans. Calcium has previously been implicated as being a global NF-κB regulator, with Ca²⁺ influx through the plasma membrane being widely held to be the main source of calcium-mediated signals. As will be appreciated by one knowledgeable in the art, the global importance of calcium levels predicts that pharmacological manipulation of calcium would be a poor choice for regulating NF-κB or for treating diseases and disorders characterized by NF-κB induction.

### SUMMARY OF THE INVENTION

The present invention provides the use of Xestospongin C in the manufacture of a pharmaceutical composition for treating or preventing a human immunodeficiency virus infection.

### BRIEF DESCRIPTION OF THE DRAWINGS

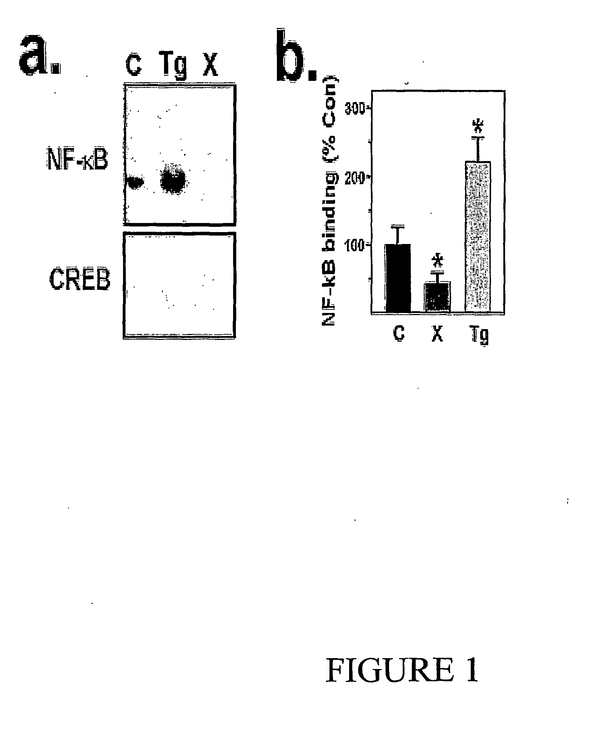

FIGURE 1 shows the effects of XeC, Tg or vehicle on basal levels of NF-κB, NF-κB binding and one-week-old cultured cerebral cortical neurons from E-18 rat embryos. a) Neurons were treated with vehicle (C), 100 nM thapsigargin (Tg) or 1 µM Xestospongin C (X). After 6 h, total cell extracts were prepared, and EMSA (electromobility shift assay) assays performed using oligonucleotides specific for NF-κB binding site. b) Autoradiographs from 4 separate EMSA assays were scanned and densitometric analyses were performed. Data are means ± S.E.M. (*p<0.05 vs. control; Anova with Scheffe's post-hoc test).

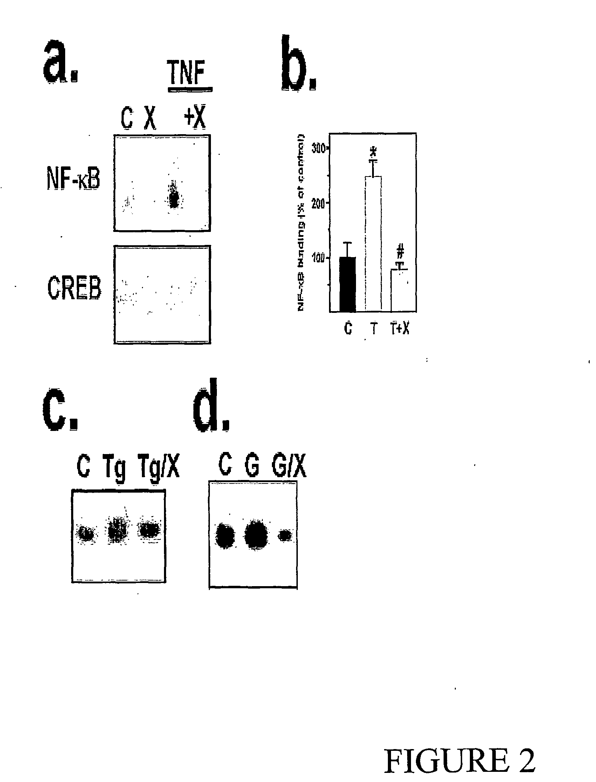

FIGURE 2 shows the results of experiments wherein following treatment of cells with either vehicle or 1 µM XeC for 30 minutes cells were incubated with TNFα, Tg, or glutamate for 6 h, total cell extracts were prepared, and EMSAs were performed using oligonucleotides specific for NF-κB or CREB binding sites. a) effects of 100 ng/ml TNFα in the absence or presence of XeC on NF-κB binding. b) Films from 3 separate EMSAs from cells treated with vehicle (C), 100 ng/ml TNFα (T), or 1 µM XeC for 30 min prior to TNFα (T + X) were scanned and densitometric analyses were performed. Data are means ± S.E.M. *p<0.05 v. control, # p<0.01 v. TNFα alone; Anova with Scheffe's post-hoc test. c) Effects of 100 nM thapsigargin in the absence (Tg) or presence (Tg/X) of 1 µM XeC on NF-κB binding. d) Effects of 20 µM glutamate in the absence (G) or presence (G/X) of 1 µM XeC on NF-κB binding.

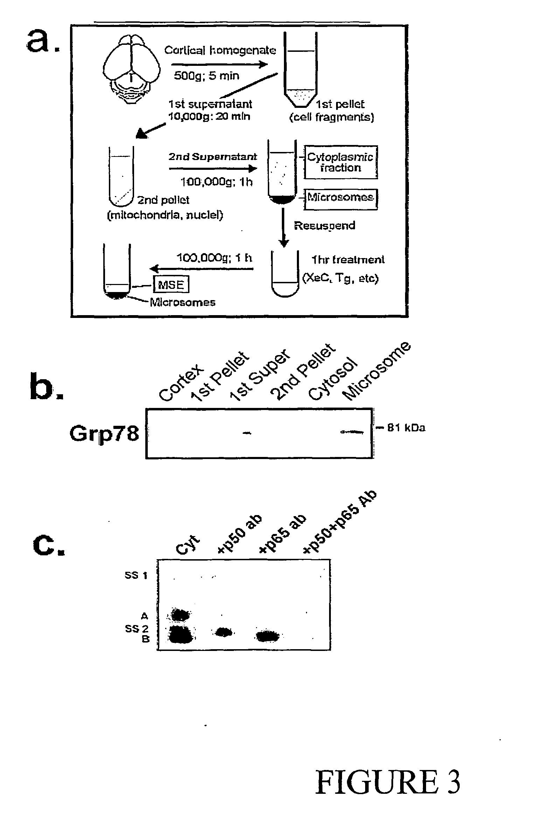

FIGURE 3 shows a) Diagrammatic representation of microsome and microsome extract (MSE) isolation technique. b) Equal amounts of protein from the subcellular fractions were separated by SDS-PAGE and immunoreacted with an antibody against an ER-resident protein Grp78. c) Supernatant fractions from brain cortex following 100,000 x g centrifugation for 1h (cytoplasmic fraction) were incubated 45 min with p50 antibody, p65 antibody, or a combination of p50 and p65 antibodies. Binding activity and band position were then analyzed using EMSA assay. The four sites of NF-κB binding identified are referred to as band A (A), band B (B), supershifted band 1 (ss1) and supershifted band 2 (ss2).

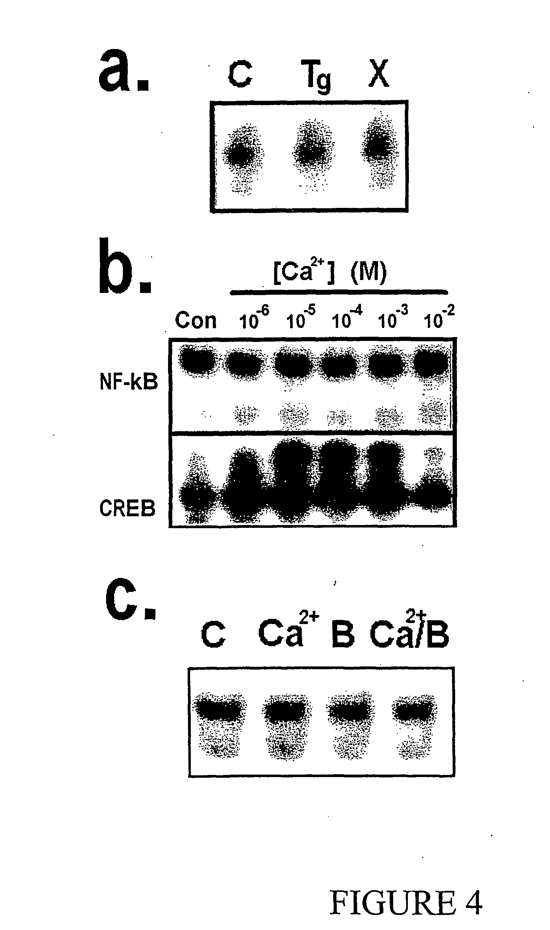

FIGURE 4 shows a) Aliquots of cytoplasmic fraction were left untreated (Cyt) or treated with 100 nM thapsigargin (Tg) or 1 µM Xestospongin C (X) for 1h, and NF-κB binding activity was determined by EMSA. b) Aliquots of cytoplasmic fractions were left untreated (Cyt) or were treated with calcium at concentrations from 10⁻⁶ to 10⁻² M for 1h, and NF-κB binding activity was determined by EMSA. c) Aliquots of cytoplasmic fraction was left untreated (Cyt) or treated for 1h with 10 µM calcium (Ca²⁺), 10 µM BAPTA-AM (B), or calcium and BAPTA-AM (Ca²⁺/B), and NF-κB binding activity was determined.

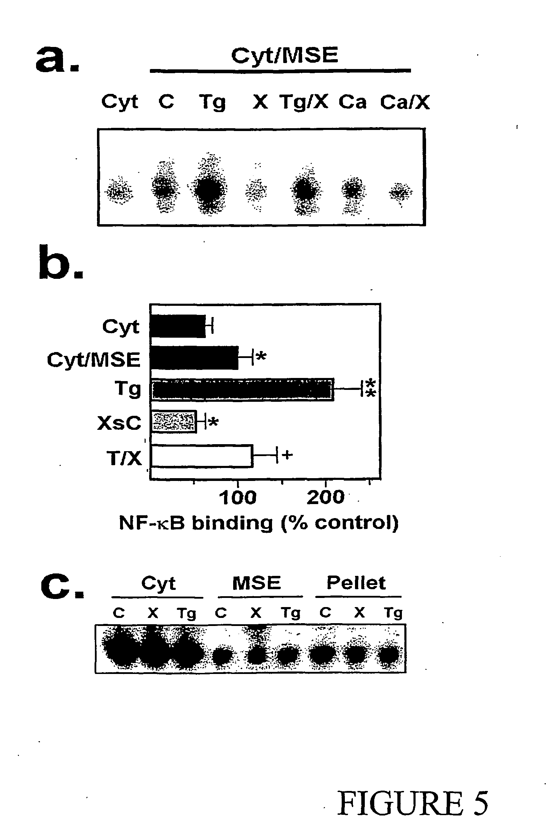

FIGURE 5 shows a) Effects of microsomal extract on NF-κB binding activity. Microsomes were pretreated for 30 min with vehicle or 1 µM XeC (X), and then incubated for 1 h with vehicle (C), 100 nM thapsigargin (Tg), or 10 µM calcium (Ca). The microsome suspension was then centrifuged at 100,000 g, and 3-5 µl microsome extract (MSE) were added to 8-10 µl of cytoplasmic fraction. After incubation for 1h at 37 °C, NF-κB binding activity was determined. As a control, the cytoplasmic fraction without addition of microsomes (Cyt) was also analyzed. b) Exposures from 4 different experiments were scanned and densitometry was performed. Data are means ± S.E.M. (*p<0.05, **p<0.01 vs. cytoplasm alone; +p<0.05 vs. thapsigargin alone; # p<0.05 vs Cyt/MSE, Anova with Scheffe's post-hoc test). c) Cytoplasmic fraction (Cyt), microsome extract (MSE), or pelleted microsomes (Pellet) were treated with vehicle (C), 1 µM XeC (X), or 100 nM thapsigargin (Tg) for 1 h prior to being analyzed for NF-κB binding activity.

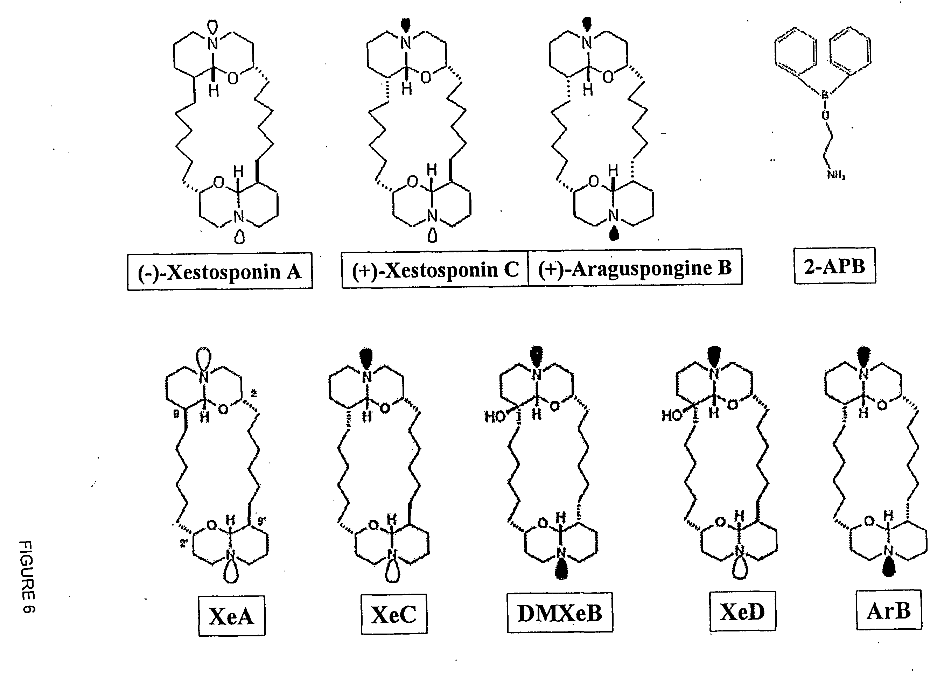

FIGURE 6 shows the chemical structures of Xestospongin A, Xestospongin C and Araguspongine B.

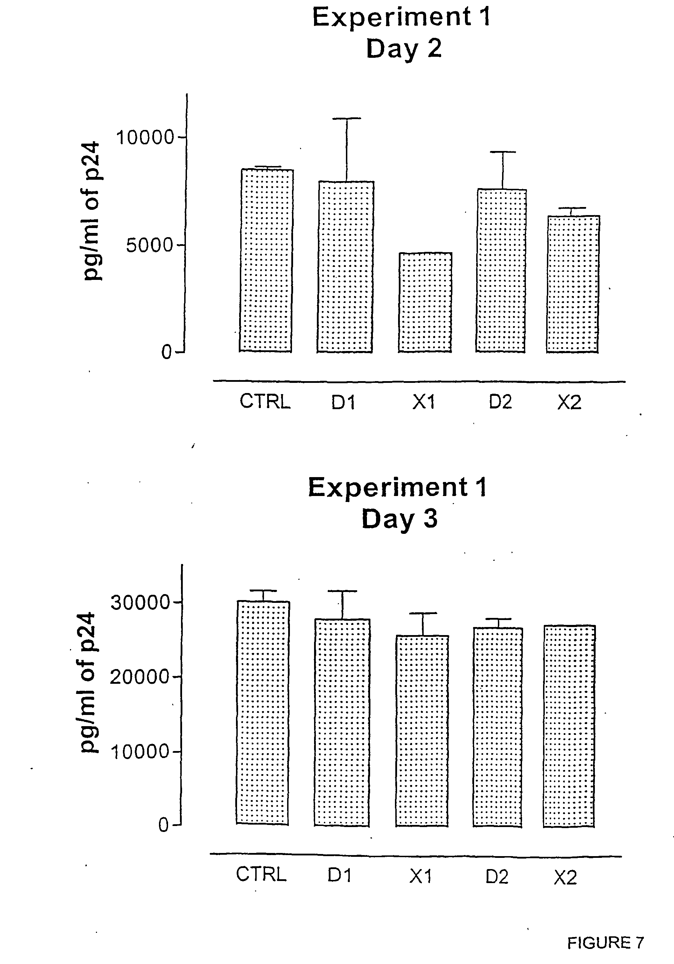

FIGURE 7 shows bar graphs of p24 levels in peripheral blood lymphocytes 2 and 3 days post-infection with HIV CSF. CTRL cells were in media only, D1 cells were treated 1 time, 2 hours pre-infection with DMSO, D2 cells were treated once 2 hours pre-infection and once 6 hours post-infection with DMSO, X1 cells were treated 1 time, 2 hours pre-infection with XeC in DMSO and X2 cells were treated once 2 hours pre-infection and once 6 hours post-infection with XeC in DMSO.

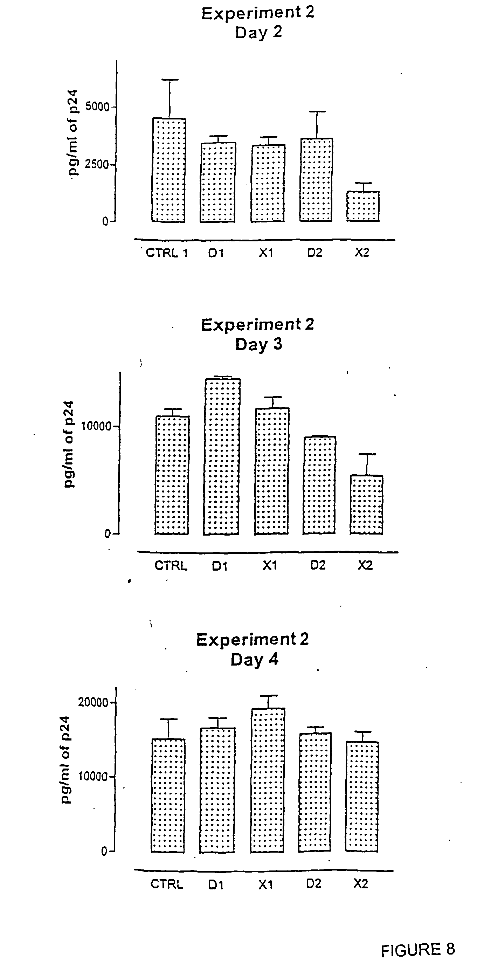

FIGURE 8 shows bar graphs of p24 levels in cultures of peripheral blood lymphocytes 2, 3 and 4 days post-infection with HIV CSF. CTRL cells were in media only, D1 cells were treated 1 time, 2 hours pre-infection with DMSO, D2 cells were treated once 2 hours pre-infection and once 6 hours post-infection with DMSO, X1 cells were treated 1 time, 2 hours pre-infection with XeC in DMSO and X2 cells were treated once 2 hours pre-infection and once 6 hours post-infection with XeC in DMSO.

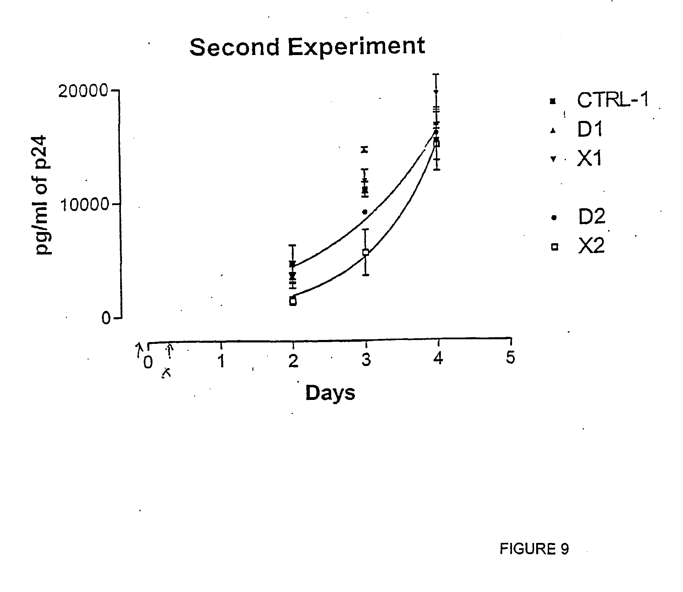

FIGURE 9 shows a graph of p24 levels in cultures of peripheral blood lymphocytes 2, 3 and 4 days post-infection with HIV CSF. CTRL cells were in media only, D1 cells were treated 1 time, 2 hours pre-infection with DMSO, D2 cells were treated once 2 hours pre-infection and once 6 hours post-infection with DMSO, X1 cells were treated 1 time, 2 hours pre-infection with XeC in DMSO and X2 cells were treated once 2 hours pre-infection and once 6 hours post-infection with XeC in DMSO.

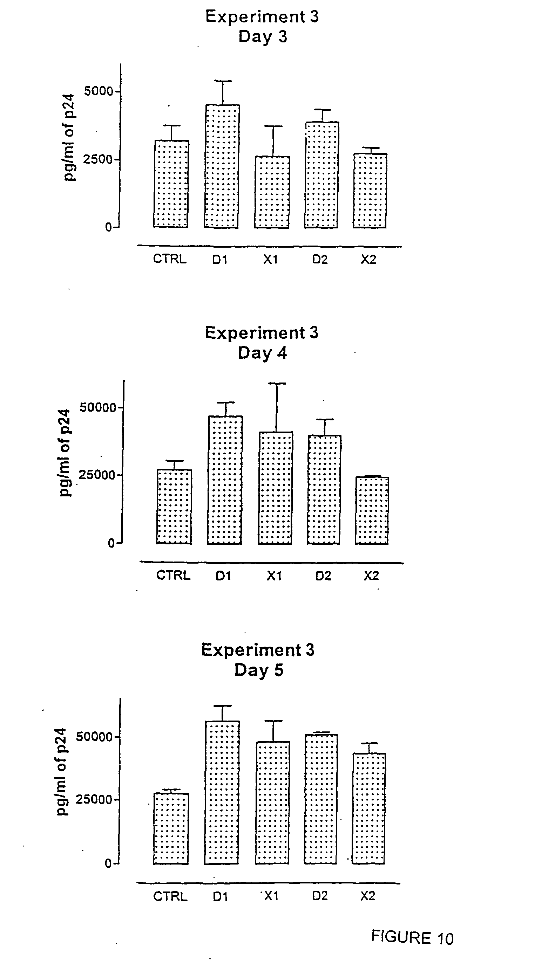

FIGURE 10 shows bar graphs of p24 levels in cultures of peripheral blood lymphocytes 3, 4 and 5 days post-infection with HIV CSF. CTRL cells were in media only, D1 cells were treated 1 time, 2 hours pre-infection with DMSO, D2 cells were treated once 2 hours pre-infection and once 6 hours post-infection with DMSO, X1 cells were treated 1 time, 2 hours pre-infection with XeC in DMSO and X2 cells were treated once 2 hours pre-infection and once 6 hours post-infection with XeC in DMSO.

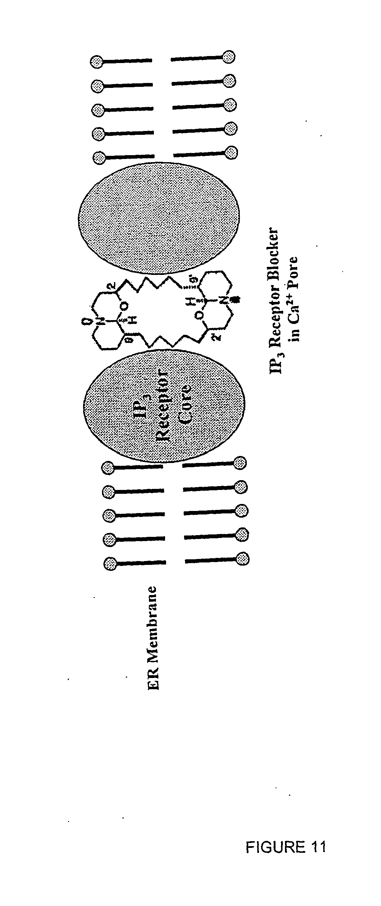

FIGURE 11 is a model of IP₃ receptor blocker in a calcium channel pore.

### DESCRIPTION OF THE PREFERRED EMBODIMENTS

Unless defined otherwise, all technical and scientific terms used herein have the same meaning as commonly understood by one of ordinary skill in the art to which the invention belongs. Although any methods and materials similar or equivalent to those described herein can be used in the practice or testing of the present invention, the preferred methods and materials are now described.

### DEFINITIONS

As used herein, "effective amount" refers to the administration of an amount of a given compound that achieves the desired effect.

As used herein, "disease characterized by IP₃ receptor mediated release of calcium" refers to diseases or disorders which require IP₃ receptor-mediated calcium release and subsequent NF-κB activation for disease progression, namely HIV.

As used herein, "viral disease" refers to diseases or disorders caused by viruses that require NF-κB activation for replication for example, HIV.

As used herein, "XeC" refers to Xestospongin C, shown in Figure 6, a compound isolated from the *Xestospongia* species.

As used herein, "IP₃ receptor-mediated calcium channel blocker" refers to compounds capable of blocking calcium release from the endoplasmic reticulum mediated by the IP₃ receptor, for example, XeC, as shown in Figure 6.

As used herein, "IP₃ receptor-mediated calcium channel modulator" refers to compounds capable of regulating IP₃ receptor-mediated calcium release, for example, IP₃ receptor-mediated calcium channel blockers.

As used herein, "purified" does not require absolute purity but is instead intended as a relative definition. For example, purification of starting material or natural material to at least one order of magnitude, preferably two or three orders of magnitude is expressly contemplated as falling within the definition of "purified".

As used herein, the term "isolated" requires that the material be removed from its original environment.

As used herein, the term "treating" in its various grammatical forms refers to preventing, curing, reversing, attenuating, alleviating, minimizing, suppressing or halting the deleterious effects of a disease state, disease progression, disease causitive agent other abnormal condition.

As discussed herein; it is important to note that the prior art teaches that it is calcium crossing the plasma membrane and entering the cytoplasm of a cell that is required for activation of NF-κB within that cell. However, it is shown herein that NF-κB activation is independent of cytoplasmic calcium levels.

As discussed below, there are several areas for which blockage of IP₃ receptor-mediated calcium release may be therapeutic. For example, as a regulator of NF-κB, it may be used for inhibiting viral diseases, as discussed herein.

As discussed herein, treatment of cell cultures with an IP₃ receptor-mediated calcium channel modulator, for example, an IP₃ receptor-mediated calcium channel blocker, for example, XeC prior to and post HIV infection resulted in reduced virus levels (based on p24 levels) compared to control cultures. That treatment with an IP₃ receptor mediated calcium channel modulator is reducing the number of viral particles present in the supernatant clearly indicates that an IP₃ receptor mediated calcium channel modulator is a potential treatment for HIV. Specifically, the IP₃ receptor mediated calcium channel modulator is reducing viral load and viral particle assembly. This in turn indicates that an IP₃ receptor mediated calcium channel modulator on its own or in combination with other treatments known in the art and discussed herein can be used to treat HIV.

Although not wishing to be bound or limited to one particular theory, the inventor believes that extracellular tat is bound by cells at a receptor. Binding of tat protein at the receptor results in a G-protein-mediated activation of phospholipase C which in turn leads to production of IP₃. IP₃ is bound by the IP₃ receptors on the ER which in turn leads to activation of NF-κB. In latent cells, induction of NF-κB leads to viral production; in uninfected cells, induction of NF-κB leads to induction of anti-stress and anti-apoptotic pathways. These pathways in effect prepare the uninfected cell for subsequent viral infection.

Thus, interfering with any stage of this pathway is a potential treatment for HIV. For example, phospholipase C inhibitors are well known in the art (see for example US Patent 5,847,074; US Patent 5,519,074; and US Patent 5,352,810) and have been proposed for treating inflammation-related disorders and cancer. However, the use of these inhibitors for treating HIV has not been proposed or considered. Specifically, inhibiting phospholipase C would in turn prevent NF-κB activation which would reduce viral load and slow spread of the virus.

Furthermore, phospholipase C is known to be activated by Gᵢ/Gₒ proteins and G_{q} proteins. As such, inhibitors of these classes of G proteins, for example, pertussis toxin and the like, will also inhibit HIV disease progression.

In addition to blocking IP₃-mediated calcium release using XeC and related compounds, it may also be possible to decrease HIV infectivity by lowering IP₃ receptor levels. It is of note that at least three isoforms of IP₃ have been identified and cloned (Furuichi et al, 1989, *Nature* **342:** 32-38; Mignery et al., 1990, *J*. *Biol. Chem.* **265:** 12678-126885; Sudhof et al., 1991, *EMBO J.* **10:** 3199-3205; Ross et al., 1992, *Proc. Natl. Acad. Sci. USA* **89:** 4265-4269; Maranto, 1994, *J. Biol. Chem.* **269**: 1222-1230; Yamada et al., 1994, *Biochem. J*. **302:** 781-790; Harnick et al., 1995, *J*. *Biol. Chem.* **270:** 2833-2840), meaning that gene replacement therapies and anti-sense probes directed against IP₃ receptors for treating HIV are within the knowledge of the art.

Other known inhibitors of IP₃ receptors include 2-aminoethoxydiphenyl borate (2-APB) (Gysembergh et al., 1999, *Am. J. Physiol.* **277:** H2458-H2469; Wilcox et al., 1998, *T.I.P.S.* **19:** 467-475), heparin (Ghosh et al., 1988, *J*. *Biol. Chem.* **253:** 11075-11079; Kobayashi et al., 1988, *Biochem. Biophys. Res. Comm.* **153:** 625-631) and a number of known monoclonal antibodies (Nakade et al., 1991, *Biochem. J.* **277:** 125-131; Sullivan et al., 1995, *Proc. Natl. Acad. Sci.* USA **92**: 8611-8615). While these compounds are less than ideal due to non-specificity (heparin) and membrane impermeability, they may prove useful treatments for HIV provided these limitations can be overcome.

Thus, the IP₃ receptor mediated calcium channel modulators, for example, IP₃ receptor antibodies, IP₃. receptor antisense probes, heparin, PLC inhibitors, IP₃ receptor mediated calcium channel blockers, G protein inhibitors and the like discussed above act to limit IP₃ receptor mediated calcium release either by blocking release, blocking the receptor, or inhibiting receptor production. As will be apparent to one knowledgeable in the art, all of these may be used as treatments for HIV.

The IP₃ receptor-mediated calcium channel blocker of the present invention is Xestospongin C, shown in Figure 6. Xestospongin A, B, C and D represent a class of macrocyclic 1-oxaquinolizidines isolated from the Australian sponge, *Xestospongia exigua* (Nakagawa et al., 1984, *Tetrahedron Letters* **25**: 3227-3230). Subsequently, nine bis-oxaquinolizidine alkaloids (Araguspongines A-J) were isolated from *Xestospongin* (Kobayashi et al., 1989, *Chem. Pharm. Bull.* **37**: 1676-1678). It has subsequently been shown that five of these compounds are potent blockers of IP₃-mediated calcium release - XeA, XeC, XeD, ArB and demethylxestospongin B (DMXeB) (Gafni et al., 1997). Gafni et al. proposed that based on the lipophillic elongated core structure with two partially charged N groups at either end, (see Figures 6, 11), the listed compounds provide ideal lipophilic/hydrophilic moieties to fit into the IP₃ receptor channel port, as shown in Figure 11. As such, any drastic changes to net charge and molecular dimensions would likely have deleterious effects on the ability of the molecules to act as IP₃ receptor blockers.

Synthetically, Xestospongin A and other related alkaloids can be derived from bis-hydroxypyridinium dimer (Baldwin et al., 1998, *J. Am. Chem. Soc*. **120**: 8559-8560).

In some embodiments discussed below, the xestospongin C is used at a dosage or concentration of 0.1 to 100 µM. In other embodiments, the dosage may be 0.1 - 10 µM or 1 to 100 µM. In yet other embodiments, the dosage may be 1 to 10 µM.

As will be apparent to one knowledgeable in the art, a therapeutically effective amount of the IP₃ receptor-mediated calcium channel modulator is the amount sufficient to achieve the desired result. For treating AIDS, the therapeutically effective amount is the amount sufficient to inhibit HIV replication and/or activation. It is well within the ability of a person skilled in the art to measure HIV activation and replication using well known markers such as T cell count, p24 assay, viral count etc. The amount administered will vary according to the concentration of the active agent and the body weight of the patient. Other factors include the degree of infection, the body weight and the age of the patient.

In some embodiments, the xestospongin C at concentrations or dosages discussed above may be combined with a pharmaceutically or pharmacologically acceptable carrier, excipient or diluent, either biodegradable or non-biodegradable. Exemplary examples of carriers include, but are by no means limited to, for example, poly(ethylene-vinyl acetate), copolymers of lactic acid and glycolic acid, poly(lactic acid), gelatin, collagen matrices, polysaccharides, poly(D,L lactide), poly(malic acid), poly(caprolactone), celluloses, albumin, starch, casein, dextran, polyesters, ethanol, mathacrylate, polyurethane, polyethylene, vinyl polymers, glycols, mixtures thereof and the like. Standard excipients include gelatin, casein, lecithin, gum acacia, cholesterol, tragacanth, stearic acid, benzalkonium chloride, calcium stearate, glyceryl monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, polyoxyethylene stearates, colloidol silicon dioxide, phosphates, sodium dodecylsulfate, carboxymethylcellulose calcium, carboxymethylcellulose sodium, methylcellulose, hydroxyethylcellulose, hydroxypropylcellulose, hydroxypropylmethycellulose phthalate, noncrystalline cellulose, magnesium aluminum silicate, triethanolamine, polyvinyl alcohol, polyvinylpyrrolidone, sugars and starches. See, for example, Remington: The Science and Practice of Pharmacy. 2000, Gennaro, AR ed., Eaton, PA: Mack Publishing Co.

As will be apparent to one knowledgeable in the art, specific carriers and carrier combinations known in the art may be selected based on their properties and release characteristics In view of the intended use. Specifically, the carrier may be pH-sensitive, thermo-sensitive, thermo-gelling, arranged for sustained release or a quick burst. In some embodiments, carriers of different classes may be used in combination for multiple effects, for example, a quick burst followed by sustained release.

In other embodiments, Xestospongin C at concentrations or dosages described above may be encapsulated for delivery. Specifically, Xestospongin C may be encapsulated in biodegradable microspheres, microcapsules, microparticles, or nanospheres. The delivery vehicles may be composed of, for example, hyaluronic acid, polyethylene glycol, poly(lactic acid), gelatin, poly(E-caprolactone), or a poly(lactic-glycolic) acid polymer. Combinations may also be used, as, for example, gelatin nanospheres may be coated with a polymer of poly(lactic-glycolic) acid. As will be apparent to one knowledgeable in the art, these and other suitable delivery vehicles may be prepared according to protocols known in the art and utilized for delivery of the xestospongin C. Alternatively, the delivery vehicle may be suspended in saline and used as a nanospray for aerosol dispersion onto an area of interest. Furthermore, the delivery vehicle may be dispersed in a gel or paste, thereby forming a nanopaste for coating a tissue or tissue portion.

It is of note that the Xestospongin C as described above may be combined with permeation enhancers known in the art for improving delivery. Examples of permeation enhancers include, but are by no means limited to those compounds described in U.S. Pat. Nos. 3,472,931; 3,527,864; 3,896,238; 3,903,256; 3,952,099; 4,046,886; 4,130,643; 4,130,667; 4,299,826; 4,335,115; 4,343,798; 4,379,454; 4,405,616; 4,746,515; 4,788,062; 4,820,720; 4,863,738; 4,863,970; and 5,378,730; British Pat. No. 1,011,949; and Idson, 1975, J. Pharm. Sci. **64**:901-924.

In some embodiments, the xestospongin C in any suitable form as described above, may be combined with biological or synthetic targetting molecules, for example, site-specific binding proteins, antibodies, lectins or ligands, for targetting the xestospongin C to a specific region or location.

In other embodiments, the xestospongin C may be combined with other known treatments a form of joint therapy. In the case of AIDS, the xestospongin C may be combined with other anti-HIV compounds, for example, azidothymidine (AZT), lamivudine (3TC), dideoxyinosine (ddi), dideoxycytidine (ddc) and ritonavir, as well as other reverse transcriptase and protease inhibitors.

As discussed above, a number of viruses have also evolved to include NF-κB binding sites within their promoters, for example, Adenovirus, Avian Leukosis Virus, Bovine Leukemia Virus, Cytomegalovirus, Epstein-Barr Virus, Hepatitis C virus, HIV, Herpes simplex virus, Polyoma virus, Measles virus, Simian immunodeficiency virus and Simian virus 40. As discussed above, it has been hypothesized that a low level of NF-κB activation is part of the mechanism by which some viruses, for example, Epstein-Barr virus, Herpes simplex virus, Cytomegalovirus or Human immunodeficiency virus maintain their chronic infections (Pahl, 1999, *Oncogene* **18**:6853-6866). This means that inhibiting NF-κB activation using an IP₃ receptor mediated calcium channel modulator would inhibit replication of these viruses, and therefore would accomplish at least one of the following: prevent or limit viral particle assembly; prevent or decrease the rate of viral replication, decreasing viral load, prevent or limit the rate of viral infection and prevent further infection by the virus.

In addition, the HIV virus produces tat, which is in turn a powerful NF-κB activator. In the context of HIV infection, it appears that NF-κB has three major roles: 1) NF-κB is one of the most important transcription factors for production of HIV proteins, and is therefore critical for viral replication; 2) NF-κB activation, as discussed above, represents an anti-apoptotic response in cells, thus allowing the cells to remain viable during the infection and replication phase of the virus; and 3) NF-κB promotes transcription of many pro-inflammatory cytokines, causing recruitment of surrounding immune cells, which may then become infected by virus particles exiting the host cell. Thus, activation of NF-κB represents a central strategy of HIV in order to aid in replication and to keep cells viable during the infection and replication stage.

Previous work has shown that tat specifically causes release of calcium from IP₃ receptor mediated stores in the endoplasmic reticulum (Mayne et al., 2000). Thus, tat may represent a specific signal shed from HIV to activate NF-κB in surrounding cells in order to prepare them for infection. That is, as discussed herein, NF-κB activation enhances stress resistance in cells, meaning that infected cells are more likely to survive. In addition, even in the absence of tat, infected cells displaying increased NF-κB binding produce large amounts of pro-inflammatory cytokines which themselves cause NF-κB activation in neighbouring cells. Furthermore, astrocytes containing virus in a latent stage can be induced to become active viral producers by exposure to pro-inflammatory cytokines which activate NF-κB. However, IP₃ receptor-mediated calcium channel modulators will inhibit the ability of tat to activate IP₃ receptor-mediated calcium release in neurons and thus prevent NF-κB activation. In addition, human macrophages exposed to an IP₃ receptor-mediated calcium channel modulator were refractory to NF-κB activation and XeC treatment inhibited pro-inflammatory cytokine production in an immune cell line. As such, IP₃ receptor-mediated calcium channel modulator-mediated inhibition of NF-κB activation would accomplish at least one of the following: decrease viability of HIV infected cells, decrease the rate of viral replication, decrease the rate of further infection of the virus and inhibit reactivation of viral replication in latent cells.

The dose administered to a patient, in the context of the present invention should be sufficient to effect a beneficial therapeutic response in the patient over time. In determining the effective amount of the IP₃ receptor-mediated calcium channel modulator xestospongin C, the dose will be determined by the condition of the patient, as well as body weight or surface area of the patient to be treated. Administration may be accomplished by a single dose or divided doses. For a typical 70 kg patient, a dose equivalent to approximately 0.1 µg to 10 mg may be administered.

The invention will now be described further by way of examples.

### EXAMPLE I - NEURONAL CELL CULTURES

Cerebral cortices were removed from embryonic day 18 Sprague-Dawley rats (Harlan, Indianapolis, IN). Cells were dissociated as described previously (Mattson et al., 1995, *J*. *Neurochem.* **65**:1740-1751.) and were seeded into polyethyleneimine-coated 60 mm culture dishes containing Eagle's Minimum Essential Medium supplemented with 26 mM NaHCO₃, 40 mM glucose, 20 mM KCl, 1 mM sodium pyruvate, 10% (v/v) heat-inactivated fetal bovine serum (Sigma, St. Louis, MO), and 0.001% gentamycin sulfate. After a 3-5 h incubation period to allow for cell attachment, the medium was replaced with 2 ml of Neurobasal Medium with B27 supplements (GIBCO Life Technologies, Grand Island, NY). Previous observations have shown that greater than 80% of the cells that attach in the first few hours after plating will survive longer than 7 days in Neurobasal Medium with no additional neurotrophic factors. Experimental treatments were performed on 7-to-9-day-old neuronal cultures in which there were approximately 5% contaminating glial cells.

### EXAMPLE II - TOTAL CELL EXTRACT PREPARATION AND ELECTROPHORETIC MOBILITY SHIFT ASSAY

Total cell extracts were prepared as described (Baeuerle and Baltimore, 1988, *Cell* **53**:211-217). Briefly, cells were harvested, washed twice with ice-cold PBS and lysed for 30 min at 4°C in Totex buffer (20 mM HEPES, pH 7.9, 350 mM NaC1, 20% glycerol, 1% NP-40, 1mM MgC1₂, 0.5 mM EDTA, 0.1 mM EGTA, 5 mM DTT, 0.1% PMSF, 1 % aprotinin, 4 µg/ml leupeptin). Samples were centrifuged at 14,000 x g for 10 min and aliquots of supernatant were collected and stored at -70°C until taken for assay. The protein content of the extract (supernatant) was measured by Bradford method (BIO-RAD Laboratories, Ercules, CA, USA). For electromobility shift assays, equal amounts of protein were incubated in a 20 µl reaction mixture containing 20 µg BSA, 1 µg poly(dl-dC), 2 µl buffer D (20 mM HEPES, pH 7.9, 20% glycerol, 100 mM KCl, 0.5 mM EDTA, 0.25% NP-40, 2 mM DTT, 0.1% PMSF), 4 µl buffer F (20% Ficoll-400, 100 mM HEPES, pH 7.9, 300 mM KCl, 10 mM DTT, 0.1% PMSF), and 20,000-50,000 cpm of ³²P-labeled oligonucleotide (Promega, Madison, WI, USA) corresponding either to a NF-κB site (5'-AGT TGA GGG GAC TTT CCC AGG C-3') or a CREB binding site (5'-AGA GAT TGC CTG ACG TCA GAG AGC TAG-3').

After 20 min at room temperature, reaction products were separated on a 7% non-denaturing polyacrylamide gel. Competition experiments were performed by incubating extracts with labeled oligonucleotide probe in the presence of 100-fold molar excess unlabeled NF-κB or CREB oligonucleotide. To characterize the NF-κB complexes, supershift assays were performed. Extracts were preincubated for 45 min at room temperature with antibodies recognizing either p50 or p65 sub-units of NF-κB (Santa Cruz Biotechnology, Santa Cruz, CA) or c-Fos (Oncogene Research Products, Cambridge, MA). The mixtures were further incubated with ³²P-labeled probe and resolved as described above. Radioactivity of dried gels was detected by exposure to Kodak X-Omat film and images on the developed film were scanned into a computer using a UMAX 1200s scanner. Densitometry was performed using Scion Image software (Scion Inc., Frederick, MD). Paint Shop Pro software (JASC Inc. Minneapolis, MN) was used for preparation of the final figures.

### EXAMPLE III - PREPARATION OF CYTOPLASMIC AND MICROSOMES FRACTIONS

Cerebral cortices from adult female Sprague-Dawley rats were homogenized (20 strokes at 300 rpm) with a Teflon homogenizer in ice-cold buffer containing 1 mM EDTA, 0.32 M sucrose, 0.1 mM dithiothreitol and 1 mM HEPES as described previously (Chen et al., 1998, *Diabetes* **47**:874-81) and cytoplasmic and microsome fractions were isolated by differential centrifugation. Briefly, tissue fragments and cellular debris were removed by centrifugation at 500 g for 10 min and supernatants were centrifuged at 20,000 g for 20 min to pellet intact mitochondria and nuclei. The supernatant was then centrifuged at 100,000 g for 1 h in order to obtain the cytoplasmic fraction and the microsomal pellet enriched in endoplasmic reticulum and golgi membranes. Microsome extracts (MSE) were prepared by suspending microsomes in 1:100 v/v ice-cold buffer and centrifuging at 100,000 x g for 1 hr. Microsomes were then re-suspended in 1:2 v/v volume buffer and treated as described below. At the end of the treatment, the suspension was centrifuged at 100,000 g for 1h, and supernatants were used as MSE. For experiments involving incubation of MSE with cytoplasmic extract, 3 µl of MSE were added to 10 µl cytoplasmic extract and mixtures were incubated at room temperature for 1h.

### EXAMPLE IV - WESTERN BLOTTING

Separation and protein quantification of grp78 by western blotting was done using methods similar to those reported previously (Cheng et al., 1995, *J. Neurochem.* **65**:2525-2536.). Proteins in various cell fractions were separated by SDS-PAGE (10% acrylamide) using the method of Laemmli (Laemmli et al., 1970, *J. Mol. Biol.* **47**:69-85). Following electrophoretic transfer to nitrocellulose, the protein was immunoreacted with a rabbit polyclonal antibody against grp78 (Stressgen Biotechnology) followed by incubation with peroxidase-labeled anti-rabbit IgG secondary antibody (1:4000). Immune blots were processed further using a chemiluminescence kit (Boehringer Mannheim) according to the manufacturer's protocol. Images of blots were scanned and quantified as described above.

### EXAMPLE V - XESTOSPONGIN C DECREASES, AND THAPSIGARGIN INCREASES, LEVELS OF NF-κB ACTIVITY

The possible involvement of endoplasmic reticulum pools of intracellular calcium regulated by IP₃ receptors in controlling basal levels of NF-κB was investigated using Xestospongin C (XeC) a specific membrane permeable modulator of IP₃ -mediated Ca²⁺ release (Gafni et al., 1997, *Neuron* **19**:723-733; Hu et al., 1999, *J*. *Biol. Chem.* **274**:33995-33998), and thapsigargin (Tg), a specific inhibitor of ER calcium/ATPase (Thastrup et al., 1990, *Proc. Natl. Acad. Sci. USA* **87**:2466-2470; Davidson and Varhol, 1995, *J. Biol. Chem.* **270**:11731-11734.). Cultured primary cortical neurons were treated with either 1 µM XeC, 100 nM Tg or vehicle for 6h and protein extracts were analyzed for NF-κB binding activity by EMSA electrophoresis. XeC caused a statistically significant (p<0.05) reduction in basal NF-κB binding activity relative to control levels, as shown in Figures 1a and 1b. Treatment with Tg, a compound that causes massive release of ER luminal calcium, produced a statistically significant (p<0.05) elevation in NF-κB activation as shown in Figures 1a and 1b. To demonstrate that the effects of XeC and Tg were specific for NF-κB, we determined the levels of CREB, a calcium-sensitive transcription factor thought to play a role in learning and neuronal survival (Stevens, 1994, *Neuron* **13**:769-770; Huang and Stevens, 1998, *Essays Biochem.* **33**:165-178). In contrast to effects on NF-κB, XeC and Tg produced slightly augmented CREB binding activity; however, this was not statistically significant (data not shown).

### EXAMPLE VI- XEC INHIBITS INDUCIBLE LEVELS OF NF-κB IN CULTURED CORTICAL NEURONS

Because NF-κB is not only a constitutively active transcription factor, but is also highly inducible in neurons, we tested the ability of XeC to inhibit NF-κB binding activity induced by the ligands TNF-α and glutamate. Cells were treated for 30 min with vehicle or XeC prior to being treated with vehicle (C) 100 ng/ml TNFα or 20 µM glutamate for 6 h. As can be seen in Figures 2a and 2b, TNFα produced a statistically significant (p<0.05) enhancement in NF-κB binding, although prior treatment with XeC completely abolished the ability of TNFα to activate NF-κB. As can be seen in Figure 2a, the effect appeared to be specific for NF-κB because neither TNFα nor XeC alone affected the binding activity of CREB. Induction of NF-κB binding activity was observed with Tg, and glutamate, and, as can be seen in Figures 2c and d, pretreatment with XeC completely abolished the elevation in NF-κB binding induced by these treatments. Cells pretreated with XeC prior to glutamate demonstrated NF-κB binding below that of control (vehicle-treated) neurons, again indicating that the decline in NF-κB binding with XeC was not directly due to reduced intracellular calcium.

### EXAMPLE VII - MICROSOMES ARE ENRICHED IN GRP 78

Because XeC, a specific blocker of calcium release from IP₃ -receptor regulated pools, could abolish NF-κB activation induced by a disparate group of agents we conducted a series of cell-free experiments to determine mechanisms involved in this ER-mediated NF-κB activation. Microsomal preparations were isolated as described (Chen et al., 1998, *Diabetes* **47**:874-81) and illustrated (fig 3a) and tests conducted in order to determine the relative enrichment of endoplasmic reticulum membranes and functionality of ER-resident calcium channels. As shown in Figure 3b, western blots of Grp78, an ER-resident heat shock protein, performed on the various fractions demonstrated that the microsomal fraction was highly enriched in Grp78 and thus ER membranes.

### EXAMPLE VIII - CHARACTERIZATION OF NF-κB COMPLEXES IN CYTOPLASMIC

### FRACTION.

To determine the identity of the major NF-κB binding complexes found in our cytoplasmic fraction, supernatants removed following 100,000 x g centrifugation were incubated with p50 antibody, p65 antibody, or a combination of p50 and p65 antibodies for 45 min. The major bands observed in these experiments were identified as band A and band B, as shown in Figure 3c. Addition of p50 antibody shifted band A in its entirety to a much higher position, called ss1, and shifted the lower band to a slightly higher position, called ss2. Band A was also apparently diminished in intensity by p50 antibody treatment. Incubation with p65 antibody, as has been previously reported (Pahl and Baeuerle, 1995), caused a disappearance of band A, but had no apparent effect on band B. Incubation with p50 and p65 antibodies caused a complete shift upward of band A to the ss 1 position, shifted band B slightly upwards, and noticeably reduced band B. Thus, band A is the p50/p65 heterodimer white band B is likely the p50/p50 homodimer. In the following experiments, band A was generally the most abundant site of NF-κB binding, while the intensity of band B varied from barely detectable to the equal of band A.

### EXAMPLE IX - MICROSOMAL EXTRACT REGULATES CYTOPLASMIC NF-κB ACTIVITY IN AN ER-CALCIUM CHANNEL-DEPENDENT MANNER

Microsomal preparations were treated with thapsigargin and calcium in the absence or presence of XeC for 1h, centrifuged, and 3 µl of supernatant (MSE) were added to 10 µl of the cytoplasmic fraction. After 1h at room temperature, samples were examined for NF-κB binding activity.

We then asked whether NF-κB could be induced by calcium directly. Aliquots of cytoplasmic fraction were treated with calcium at concentrations ranging from 1 µM to 10 mM for 1h, followed by analysis of NF-κB binding by EMSA. As shown in Figure 4b, none of the calcium concentrations used increased NF-κB binding activity. These same extracts analyzed for CREB binding activity showed that calcium concentrations of 10 µM, 100 µM, and 1 mM greatly enhanced CREB binding, probably through CaM kinase activation. These data clearly demonstrate that while the elevation of calcium in cytoplasm by itself is able to induce CREB it is not sufficient to cause NF-κB activation. Conversely, it is also true that chelation of calcium does not produce a decline in binding activity. Aliquots of cytoplasmic fraction were treated for 1h with 10 µM calcium, 10 µM BAPTA-AM, or a combination of the two. As shown in Figure 4c, 10 µM BAPTA-AM alone or in combination with calcium had no affect on the levels of the two major bands of NF-κB binding activity present in untreated cytoplasm.

### EXAMPLE X - EFFECT OF XeC ON PERIPHERAL BLOOD LYMPHOCYTES

Peripheral blood lymphocytes, previously stimulated for 72 hours with PHA, were divided into 3 groups, designated T-HIV (RPMI-10 media containing 1 µM XeC and 0.1% DMSO), N-HIV (RPMI-10 media containing 0.1% DMSO) and HIV (RPMI-10 media). The cells were then incubated in the dark at 37°C for 1 hour. The cells were then infected with a macrophage-tropic CCR5-utilizing HIV CSF for 3 hours at 37° and then washed to remove excess virus. All groups were cultured in RPMI-10% FCS + 10U/ml IL-2. Referring to Figures 7-11, cells were incubated at 37°C in 5% CO₂. At stated time points shown in the aforementioned figures, spent media was removed and replace with fresh media. The media was tested for the presence of p24 antigen as a surrogate for HIV infection and replication. As can be seen from the data, there is inhibition of Xestospongin relative to DMSO. The significance of using the CCR5 utilizing macrophage-tropic virus JR-CSF is that this is the type of virus generally transmitted sexually.

Referring to Figure 7, the media-only control (CTRL) and the DMSO-treated cells (D1 and D2) show similar levels of p24 on both days 2 and 3. However, samples X2 (treated with XeC 2 hours pre-infection and 6 hours post-infection) and X1 (treated with XeC 2 hours pre-infection) show reduced levels of p24, approximately 80% and 50% of controls on day 2. However, the p24 levels of X1 and X2 increased to approximately control levels by day 3.

Referring to Figure 8, in a subsequent experiment, X2 samples showed greatly reduced p24 levels on days 2 and 3 while no effect was seen in the X1 samples. The kinetics of the data shown in Figure 8 can also be seen in Figure 9, wherein the levels of p24 in X2 is below the control levels on day 2 but increase to near wild-type levels by day 4.

Figure 10 summarizes data from a third experiment wherein p24 levels in X1 and X2 were both reduced relative to D1 and D2 on day 3. By day 4, p24 levels in X1 had increased to near control levels, but p24 levels in X2 samples remained approximately 50% of the DMSO controls.

### EXAMPLE XI - DISCUSSION

As discussed above, a wide range of conditions and ligands have been shown to activate the transcription factor NF-κB. Conditions that induce, and ligands that are associated with, cell stress responses including elevated levels of intracellular calcium, trophic factor withdrawal, oxidative stress and exposure to ultraviolet light as well as activation of cell surface receptors by cytokines such as TNF-α and interleukins have been shown to enhance the binding and/or transcriptional activity of NF-κB (Pahl, 1999, *Oncogene* **18**:6853-6866). Some have suggested that increased levels of intracellular calcium may be an underlying factor common to such activation of NF-κB (Ginn-Pease and Whisler, 1998). Specifically, it has been suggested that release of intracellular stores of calcium may play a major role in activation of NF-κB (Pahl and Baeuerle, 1996, *FEBS Lett.* **392**:129-136; Quinlan et al., 1999, *J*. *lmmunol.* **163**:5656-5665; Sen et al., 1996, *FEBS Lett.* **385**:58-62.). To help elucidate the relationship between ER calcium release, through IP₃ receptors specifically, and NF-κB activation we conducted a series of experiments and found that a) XeC reduced both basal and inducible NF-κB binding activity in cortical neurons, and b) contrary to the teachings of the prior art, this decline was independent of changes in intracellular calcium. Thus our data indicates that it is not the calcium release from ER stores that activates cytoplasmic NF-κB, but rather the decrease in intraluminal calcium.

We have demonstrated that a dose of XeC reported to be highly specific for blocking ER IP₃ calcium channels (Gafni et al., 1997, Hu et al 1999) can inhibit basal levels of NF-κB binding. XeC also significantly inhibited the ability of TNFα to induce NF-κB. Glutamate, which causes a large influx of calcium in neurons, also greatly induced NF-κB binding activity. XeC was able to totally abolish this induction, although XeC treatment did not erase the increase in [Ca²⁺]ᵢ caused by glutamate (data not shown). Glutamate induces activation of IP₃ receptors both by increasing intracellular calcium (Kato and Rubel, 1999, *J. Neurophysiol.* **81**:1587-1596; Nakamura et al., 1999, *Neuron* **24**:727-737) and by induction of IP₃ production through phospholipase C (PLC) (Liu et al., 1997, *Eur. J. Pharmacol.* **338**:277-287; Recasens and Vignes, 1995, *Ann. N. Y. Acad. Sci.* **757**:418-29). Therefore, all inducers of NFκB tested here have in common the ability to initiate calcium release through ER IP₃ receptors, either through increasing intracellular calcium, activating production of IP₃, or both. In the case of glutamate, an increase in intracellular calcium was not able to activate NFκB when IP₃ receptors were blocked with XeC.

The fact that neither calcium nor BAPTA-AM were able to change NFκB binding activity shows that the calcium released from the microsomes does not regulate NFκB. Indeed, the role calcium plays in this paradigm seems to be as an intraluminal signal. This is supported by the observation that direct activation of IP₃ receptors on microsomes using IP₃ itself led to MSE which had NF-κB stimulating properties similar to that seen with Tg. In addition, pretreatment of microsomes with XeC completely abolished this effect. Microsomes pretreated with XeC followed by calcium produced no NF-κB stimulating activity, indicating that though calcium can directly modulate ER calcium release, it has no effect when IP₃ receptors are blocked.

As discussed above, many studies implicate calcium as having a central role in governing NF-κB activity. We have shown in our *in vitro* studies that calcium per se did not affect NF-κB binding. However, our cell-free system only examined the NF-κB signaling pathway from the ER to NF-κB activation. Indeed, in the whole cell, our hypothesis that it is the filling state of the ER IP₃ pool which signals NFκB activation would tend to implicate calcium as a major upstream effector. However, both IP₃ and ryanodine receptors are acutely sensitive to [Ca²⁺]ᵢ, as is the ER resident calcium ATPase. In addition, IP₃ synthesis is sensitive to calcium levels (del Rio et al., 1994, *J. Neurochem.* **63**:535-43; Kim et al., 1999, *J*. *Biol. Chem.* **274**:26127-26134). In fact, in the absence of calcium, PLC-mediated production of IP₃ drops precipitously (Hughes and Putney, 1990, *Environ. Health Perspect.* **84**:141-147). As ER IP₃ receptors are primarily regulated by IP₃ and calcium, additional of calcium chelators will greatly reduce the activity of IP₃ receptors due to the fact that both mediators of channel opening are diminished. In addition, BAPTA-AM specifically interferes with IP₃ binding to the receptor (Taylor and Broad, 1998, *Trends Pharmacol. Sci.* **19**:370-375) and this information taken as a whole could explain a great deal of what has been observed regarding calcium regulation of NF-κB binding. The ER-overload response, examined extensively by Pahl et al., has been shown to require release of calcium from ER. Therefore, many of the most commonly used activators of NF-κB impinge upon the endoplasmic reticulum, through elevations in intracellular calcium, activation of PLC to produce IP₃, or both.

Activation of NF-κB has been implicated in a large and growing number of physiological events, many of them associated with exacerbation of pathological states. For example, overactivation of NF-κB is associated with HIV infection (Swingler et al., 1994, *Biochem. Biophys. Res*. *Commun.* **203**:623-630).

## Claims

1. Use of Xestospongin C in the manufacture of a pharmaceutical composition for treating or preventing a human immunodeficiency virus infection.

2. The use according to claim 1 wherein the Xestospongin is present in the pharmaceutical composition at a concentration of 0.1 to 100 µM.

3. The use according to claim 1 wherein the Xestospongin is present in the pharmaceutical composition at a concentration of 0.1 to 10 µM.

4. The use according to claim 1 wherein the Xestospongin is present in the pharmaceutical composition at a concentration of 1 to 100 µM.

5. The use according to claim 1 wherein the Xestospongin is present in the pharmaceutical composition at a concentration of 1 to 10 µM.

## Patentansprüche

1. Verwendung von Xestospongin C bei der Herstellung einer Arzneimittelzusammensetzung zum Behandeln oder Verhindern einer HIV-Infektion.

2. Verwendung nach Anspruch 1, bei welcher das Xestospongin in der Arzneimittelzusammensetzung in einer Konzentration von 0,1 bis 100 µM vorhanden ist.

3. Verwendung nach Anspruch 1, bei welcher das Xestospongin in der Arzneimittelzusammensetzung in einer Konzentration von 0,1 bis 10 µM vorhanden ist.

4. Verwendung nach Anspruch 1, bei welcher das Xestospongin in der Arzneimittelzusammensetzung in einer Konzentration von 1 bis 100 µM vorhanden ist.

5. Verwendung nach Anspruch 1, bei welcher das Xestospongin in der Arzneimittelzusammensetzung in einer Konzentration von 1 bis 10 µM vorhanden ist.

## Revendications

1. Application de la xestospongine C à la fabrication d'une composition pharmaceutique pour le traitement ou la prévention d'une infection par le virus de l'immunodéficience humaine.

2. Application selon la revendication 1, dans laquelle la xestospongine est présente dans la composition pharmaceutique à une concentration de 0,1 à 100 µM.

3. Application selon la revendication 1, dans laquelle la xestospongine est présente dans la composition pharmaceutique à une concentration de 0,1 à 10 µM.

4. Application selon la revendication 1, dans laquelle la xestospongine est présente dans la composition pharmaceutique à une concentration de 1 à 100 µM.

5. Application selon la revendication 1, dans laquelle la xestospongine est présente dans la composition pharmaceutique à une concentration de 1 à 10 µM.
